# EUROPEAN PATENT SPECIFICATION

(11) **EP 1 552 022 B1**
(45) Date of publication and mention of the grant of the patent: **23.01.2013**
(21) Application number: 03741986.8
(22) Date of filing: 12.06.2003
(51) Int. Cl.: C12Q 1/70, C12Q 1/68, C07H 21/04

(54) **IDENTIFICATION OF OLIGONUCLEOTIDES FOR THE CAPTURE, DETECTION AND QUANTITATION OF HEPATITIS A VIRAL NUCLEIC ACID**
IDENTIFIZIERUNG VON OLIGONUKLEOTIDEN ZUM ERFASSSEN, ZUM NACHWEIS UND ZUR QUANTIFIZIERUNG EINER HEPATITIS-A-VIRUSNUKLEINSÄURE
IDENTIFICATION DE OLIGONUCLEOTIDES A DES FINS DE CAPTURE, DETECTION ET QUANTIFICATION D'ACIDES NUCLEIQUES DU VIRUS DE L'HEPATITE A

(30) Priority: 12.06.2002 US 388544 P
(43) Date of publication of application: 13.07.2005
(73) Proprietor: Novartis Vaccines and Diagnostics, Inc., Emeryville, CA 94608 (US)
(72) Inventor: SHYAMALA, Venkatakrishna, c/o Chiron Corporation, Emeryville, CA 94662-8097 (US)
(74) Representative: Marshall, Cameron John
(86) International application number: PCT/US2003/018827
(87) International publication number: WO 2003/106641

(56) References cited:
- WO-A-91/11534
- WO-A1-91/14788
- WO-A2-01/83814
- US-A- 5 702 891
- DATABASE NCBI 5 February 1994 (1994-02-05), XP002396774 Database accession no. S66965
- DATABASE NCBI 12 April 2001 (2001-04-12), XP002396775 Database accession no. K01307
- DATABASE NCBI 27 August 1999 (1999-08-27), XP002396776 Database accession no. AJ232747
- FUJIWARA K. ET AL: 'PCR-SSCP analysis of 5'-nontranslated region of hepatitis A viral RNA: comparison with clinicopathological features of hepatitis A' DIGESTIVE DISEASES AND SCIENCES vol. 45, no. 12, December 2000, pages 2422 - 2427, XP008034195
- DIU A ET AL: "TCR BETA V BETA 9=T-CELL RECEPTOR BETA CHAIN VARIABLE REGION {JUNCTION} [HUMAN, PRIMARY BILIARY CIRRHOSIS, LIVER-INFILTRATING", GENBANK, XP002396774,

## Description

### Technical Field

The present invention pertains generally to viral diagnostics. In particular, the invention relates to nucleic acid-based assays for accurately diagnosing hepatitis A infection and detecting hepatitis A in a biological sample.

### Background Of The Invention

Hepatitis A is an enterically transmitted disease that causes fever, malaise, anorexia, nausea, abdominal discomfort and jaundice. The etiologic agent of hepatitis A, the hepatitis A virus, is a small, nonenveloped, spherical virus classified in the genus Hepatovirus of the Picomaviridae family. The HAV genome consists of a single-strand, linear, 7.5 kb RNA molecule encoding a polyprotein precursor that is processed to yield the structural proteins and enzymatic activities required for viral replication (Najarian et al., Proc. Natl. Acad. Sci. USA 82:2627-2632 (1985)). HAV grows poorly in cell culture, is not cytopathic, and produces low yields of virus. Although HAV RNA extracted from virions is infectious in cell culture (Locarnini et al., J. Virol. 37:216-225 (1981) and Siegl et al., J. Gen. Virol. 57:331-341 (1981)), direct manipulation of the viral genome becomes difficult because of its RNA composition.

HAV encodes four capsid proteins (A, B, C and D) which contain the major antigenic domains recognized by antibodies of infected individuals. In addition to the capsid proteins, antigenic domains have been reported in nonstructural proteins such as 2A and the viral encoded protease. Another important HAV antigenic domain has been described in the junction between the capsid precursor P1 and 2A.

HAV is normally acquired by the fecal-oral route, by either person-to-person contact or ingestion of contaminated food or water. However, there is the potential for HAV transmission by pooled plasma products. The absence of a lipid envelope makes HAV very resistant to physicochemical inactivation, and the virus can withstand conventional heat treatment of blood products. Thus, HAV, as well as Parvovirus B19, have been transmitted through the administration of pooled plasma derivatives. The development of sensitive and specific diagnostic assays to identify HAV antigens and/or antibodies in infected individuals as well as nucleic acid-based tests to detect viremic samples to exclude them from transfusion represents an important public health challenge.

U.S. Patent No. 5,290,677 to Robertson et al.*,* describes the capture of whole HAV virus using antibodies. RNA is isolated, and cDNA generated. The cDNA is then amplified by PCR using primers from the VP1 and VP3 capsid region of HAV genome, and the amplified product is detected using probes from the same region of the genome. The selection of the primers and probes is based on the genotype of HAV to be detected.

There remains a need for the development of reliable diagnostic tests to detect hepatitis A virus in viremic samples, in order to prevent transmission of the virus through blood and plasma derivatives or by close personal contact.

### Summary of the Invention

The present invention is based on the development of a sensitive, reliable nucleic acid-based diagnostic test for the detection of HAV in biological samples from potentially infected individuals. The techniques described herein utilize extracted sample nucleic acid as a template for amplification of conserved genomic regions of the HAV sequence using PCR, transcription-mediated amplification (TMA), as well as in a 5' nuclease assay, such as the TaqMan^{™} technique. The methods allow for the detection of HAV in viremic samples. In certain embodiments, the subject invention uses primers and probes derived from the 5' UTR region of the HAV genome. Moreover, the methods allow for a one-pot analysis wherein captured sample nucleic acids can be subjected to amplification and detection in the same container. Using the methods of the invention, infected samples can be identified and excluded from transfusion, as well as from the preparation of blood derivatives.

Accordingly, in one embodiment, the subject invention is directed to a method of detecting HAV infection in a biological sample. The method comprises:
isolating nucleic acids from a biological sample suspected of containing HAV, wherein the nucleic acids are isolated from the biological sample by a method comprising:
   i. contacting a solid support comprising capture nucleic acids associated therewith with a biological sample under hybridizing conditions wherein target nucleic acid strands hybridize with the capture nucleic acids; and
   ii. separating the solid support from the sample,
      and wherein the capture nucleic acids comprise one or more oligonucleotides, wherein each of the oligonucleotides is not more than about 60 nucleotides in length and comprises at least 10 contiguous nucleotides from a sequence selected from the group consisting of SEQ ID NO:10, SEQ ID NO:11, SEQ ID NO:12, SEQ ID NO:13 and SEQ ID NO:14;
      wherein the solid support comprises beads;
amplifying the isolated nucleic acids using at least two primers derived from the 5' UTR of the HAV genome,
   wherein each of the primers is from 10 to 60 nucleotides in length and is sufficiently complementary to a portion of the sense and antisense strands, respectively, of the isolated nucleic acid to hybridize therewith,
   and wherein
   (a) one of the primers comprises a nucleotide sequence of at least 10 contiguous nucleotides from SEQ ID NO:1 and the other primer comprises a nucleotide sequence of at least 10 contiguous nucleotides from SEQ ID NO:2, or
   (b) the primers have 90% sequence identity to the nucleotide sequences
   described in (a); and
detecting the presence of the amplified nucleic acids as an indication of the presence or absence of HAV in the sample.

In certain embodiments, the beads are magnetic beads.

In certain embodiments, the isolating, amplifying and detecting are performed in a single container.

Preferably, the capture nucleic acids further comprise a homopolymer chain at either the 3' or 5' end of about 15-25 nucleotides in length, wherein the homopolymer chain is selected from the group consisting of polyA, polyT, polyG, polyC, and polyU.

More preferably, the homopolymer chain is a polyA chain.

In certain embodiments, amplifying comprises PCR, transcription-mediated amplification (TMA) or TaqMan.

Preferably amplifying comprises TMA.

Preferably said amplifying methods, further comprise using a probe oligonucleotide comprising a detectable label for detecting the amplified sequence, wherein the probe oligonucleotide is not more than about 60 nucleotides in length and comprises at least 10 contiguous nucleotides comprising SEQ ID NO:3.

Preferably, the probe comprises detectable labels at the 5'-end and at the 3'-end.

Preferably, the detectable label is a fluorescent label selected from the group consisting of 6-carboxyfluorescein (6-FAM), tetramethyl rhodamine (TAMRA), and 2', 4', 5', 7',- tetrachloro -4-7- dichlorofluorescein (TET).

In a further embodiment, the invention is directed to a kit for detecting Hepatitis A virus (HAV) infection in a biological sample, the kit comprising:
capture nucleic acids comprising one or more oligonucleotides, wherein each of the oligonucleotides is not more than about 60 nucleotides in length and comprises a nucleotide sequence of at least 10 contiguous nucleotides of a sequence selected from the group consisting of SEQ ID NO:10, SEQ ID NO:11, SEQ ID NO:12, SEQ ID NO:13 and SEQ ID NO:14;
at least two primers wherein (a) each of the primers is not more than about 60 nucleotides in length and one primer comprises a nucleotide sequence of at least 10 contiguous nucleotides from SEQ ID NO:1 and the other primer comprises a nucleotide sequence of at least 10 contiguous nucleotides from SEQ ID NO:2;
written instructions for identifying HAV infection; and
a solid support comprising beads.

The kit preferably further comprises a polymerase and buffers.

The kit preferably further comprises a probe oligonucleotide of not more than about 60 nucleotides in length and at least 10 contiguous nucleotides comprising SEQ ID NO:3.

Prederably the probe further comprises detectable labels at the 5'-end and at the 3'-end.

Preferably the detectable label is a fluorescent label selected from the group consisting of 6-carboxyfluorescein (6-FAM), tetramethyl rhodamine (TAMRA), and 2', 4', 5', 7',- tetrachloro -4-7- dichlorofluorescein (TET).

These and other aspects of the present invention will become evident upon reference to the following detailed description and attached drawings. In addition, various references are set forth herein which describe in more detail certain procedures or compositions.

### Brief Description of the Drawings

Figures 1A-1B (SEQ ID NOS:1 and 2) depict exemplary primers for use in the amplification of the isolated HAV nucleic acids.
Figure 2 (SEQ ID NO:3) depicts a probe for use in detecting the presence of the amplified target oligonucleotides indicating the presence of HAV, where X is 6-FAM (fluorescein), and Z is a linker plus TAMRA (tetramethylrhodamine).
Figures 3A-3F (SEQ ID NOS:10-15), depict exemplary capture oligonucleotides for isolating HAV nucleic acids from a biological sample.
Figure 4A depicts an HAV wild-type target sequence (SEQ ID NO:16). Figure 4B (SEQ ID NO:17) depicts an exemplary internal control sequence for use as a control for target capture and amplification. The bolded bases represent the sequence in the wild-type that is replaced in the internal control sequence.

### Detailed Description of the Invention

The practice of the present invention will employ, unless otherwise indicated, conventional methods of chemistry, biochemistry, recombinant DNA techniques and virology, within the skill of the art. Such techniques are explained fully in the literature. See, e.g., Fundamental Virology, 2nd Edition, vol. I & II (B.N. Fields and D.M. Knipe, eds.); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Oligonucleotide Synthesis (N. Gait, ed., 1984); A Practical Guide to Molecular Cloning (1984).

It must be noted that, as used in this specification and the appended claims, the singular forms "a", "an" and "the" include plural referents unless the content clearly dictates otherwise. Thus, for example, reference to "an oligonucleotide" includes a mixture of two or more oligonucleotides, and the like.

The following amino acid abbreviations are used throughout the text:

| | |
|---|---|
| Alanine: Ala (A) | Arginine: Arg (R) |
| Asparagine: Asn (N) | Aspartic acid: Asp (D) |
| Cysteine: Cys (C) | Glutamine: Gln (Q) |
| Glutamic acid: Glu (E) | Glycine: Gly (G) |
| Histidine: His (H) | Isoleucine: Ile (I) |
| Leucine: Leu (L) | Lysine: Lys (K) |
| Methionine: Met (M) | Phenylalanine: Phe (F) |
| Proline: Pro (P) | Serine: Ser (S) |
| Threonine: Thr (T) | Tryptophan: Trp (W) |
| Tyrosine: Tyr (Y) | Valine: Val (V) |

### I. Definitions

In describing the present invention, the following terms will be employed, and are intended to be defined as indicated below.

The terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to a protein which includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts which produce the proteins or errors due to PCR amplification.

By "isolated" is meant, when referring to a polypeptide, that the indicated molecule is separate and discrete from the whole organism with which the molecule is found in nature or is present in the substantial absence of other biological macro-molecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

A polynucleotide "derived from" or "specific for" a designated sequence refers to a polynucleotide sequence which comprises a contiguous sequence of approximately at least about 6 nucleotides, preferably at least about 8 nucleotides, more preferably at least about 10-12 nucleotides, and even more preferably at least about 15-20 nucleotides corresponding, i.e., identical or complementary to, a region of the designated nucleotide sequence. The derived polynucleotide will not necessarily be derived physically from the nucleotide sequence of interest, but may be generated in any manner, including, but not limited to, chemical synthesis, replication, reverse transcription or transcription, which is based on the information provided by the sequence of bases in the region(s) from which the polynucleotide is derived. As such, it may represent either a sense or an antisense orientation of the original polynucleotide.

"Homology" refers to the percent similarity between two polynucleotide or two polypeptide moieties. Two nucleic acid, or two polypeptide sequences are "substantially homologous" to each other when the sequences exhibit at least about 50% , preferably at least about 75%, more preferably at least about 80%-85%, preferably at least about 90%, and most preferably at least about 95%-98% sequence similarity over a defined length of the molecules. As used herein, substantially homologous also refers to sequences showing complete identity to the specified nucleic acid or polypeptide sequence.

In general, "identity" refers to an exact nucleotide-to-nucleotide or amino acid-to-amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100.

Readily available computer programs can be used to aid in the analysis of homology and identity, such as ALIGN, Dayhoff, M.O. in Atlas of Protein Sequence and Structure M.O. Dayhoff ed., 5 Suppl. 3:353-358, National biomedical Research Foundation, Washington, DC, which adapts the local homology algorithm of Smith and Waterman Advances in Appl. Math. 2:482-489, 1981 for peptide analysis. Programs for determining nucleotide sequence homology are available in the Wisconsin Sequence Analysis Package, Version 8 (available from Genetics Computer Group, Madison, WI) for example, the BESTFIT, FASTA and GAP programs, which also rely on the Smith and Waterman algorithm. These programs are readily utilized with the default parameters recommended by the manufacturer and described in the Wisconsin Sequence Analysis Package referred to above. For example, percent homology of a particular nucleotide sequence to a reference sequence can be determined using the homology algorithm of Smith and Waterman with a default scoring table and a gap penalty of six nucleotide positions.

Another method of establishing percent homology in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence homology." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-bin/BLAST.

Alternatively, homology can be determined by hybridization of polynucleotides under conditions which form stable duplexes between homologous regions, followed by digestion with single-stranded-specific nuclease(s), and size determination of the digested fragments. Nucleic acid sequences that are substantially homologous can be identified in a Southern hybridization experiment under, for example, stringent conditions, as defined for that particular system. Defining appropriate hybridization conditions is within the skill of the art. See, e.g., Sambrook et al., *supra; DNA Cloning, supra; Nucleic Acid Hybridization, supra.*

"Recombinant" as used herein to describe a nucleic acid molecule means a polynucleotide of genomic, cDNA, viral, semisynthetic, or synthetic origin which, by virtue of its origin or manipulation is not associated with all or a portion of the polynucleotide with which it is associated in nature. The term "recombinant" as used with respect to a protein or polypeptide means a polypeptide produced by expression of a recombinant polynucleotide. In general, the gene of interest is cloned and then expressed in transformed organisms, as described further below. The host organism expresses the foreign gene to produce the protein under expression conditions.

A "control element" refers to a polynucleotide sequence which aids in the transcription and/or translation of a nucleotide sequence to which it is linked. The term includes promoters, transcription termination sequences, upstream regulatory domains, polyadenylation signals, untranslated regions, including 5'-UTRs and 3'-UTRs and when appropriate, leader sequences and enhancers, which collectively provide for the transcription and translation of a coding sequence in a host cell.

A "promoter" as used herein is a regulatory region capable of binding a polymerase and initiating transcription of a downstream (3' direction) nucleotide sequence operably linked thereto. For purposes of the present invention, a promoter sequence includes the minimum number of bases or elements necessary to initiate transcription of a sequence of interest at levels detectable above background. Within the promoter sequence is a transcription initiation site, as well as protein binding domains (consensus sequences) responsible for the binding of RNA or DNA polymerase. For example, promoter may be a nucleic acid sequence that is recognized by a DNA-dependent RNA polymerase ("transcriptase") as a signal to bind to the nucleic acid and begin the transcription ofRNA at a specific site. For binding, such transcriptases generally require DNA which is double-stranded in the portion comprising the promoter sequence and its complement; the template portion (sequence to be transcribed) need not be double-stranded. Individual DNA-dependent RNA polymerases recognize a variety of different promoter sequences which can vary markedly in their efficiency in promoting transcription. When an RNA polymerase binds to a promoter sequence to initiate transcription, that promoter sequence is not part of the sequence transcribed. Thus, the RNA transcripts produced thereby will not include that sequence.

A control sequence "directs the transcription" of a nucleotide sequence when RNA or DNA polymerase will bind the promoter sequence and transcribe the adjacent sequence.

A "DNA-dependent DNA polymerase" is an enzyme that synthesizes a complementary DNA copy from a DNA template. Examples are DNA polymerase I from *E. coli* and bacteriophage T7 DNA polymerase. All known DNA-dependent DNA polymerases require a complementary primer to initiate synthesis. Under suitable conditions, a DNA-dependent DNA polymerase may synthesize a complementary DNA copy from an RNA template.

A "DNA-dependent RNA polymerase" or a "transcriptase" is an enzyme that synthesizes multiple RNA copies from a double-stranded or partially-double stranded DNA molecule having a (usually double-stranded) promoter sequence. The RNA molecules ("transcripts") are synthesized in the 5' to 3' direction beginning at a specific position just downstream of the promoter. Examples of transcriptases are the DNA-dependent RNA polymerase from *E*. *coli* and bacteriophages T7, T3, and SP6.

An "RNA-dependent DNA polymerase" or "reverse transcriptase" is an enzyme that synthesizes a complementary DNA copy from an RNA template. All known reverse transcriptases also have the ability to make a complementary DNA copy from a DNA template; thus, they are both RNA- and DNA-dependent DNA polymerases. A primer is required to initiate synthesis with both RNA and DNA templates.

"RNAse H" is an enzyme that degrades the RNA portion of an RNA:DNA duplex. These enzymes may be endonucleases or exonucleases. Most reverse transcriptase enzymes normally contain an RNAse H activity in addition to their polymerase activity. However, other sources of the RNAse H are available without an associated polymerase activity. The degradation may result in separation of RNA from a RNA:DNA complex. Alternatively, the RNAse H may simply cut the RNA at various locations such that portions of the RNA melt off or permit enzymes to unwind portions of the RNA.

The terms "polynucleotide," "oligonucleotide," "nucleic acid" and "nucleic acid molecule" are used herein to include a polymeric form of nucleotides of any length, either ribonucleotides or deoxyribonucleotides. This term refers only to the primary structure of the molecule. Thus, the term includes triple-, double- and single-stranded DNA, as well as triple-, double- and single-stranded RNA. It also includes modifications, such as by methylation and/or by capping, and unmodified forms of the polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), any other type of polynucleotide which is an Nor C-glycoside of a purine or pyrimidine base, and other polymers containing nonnucleotidic backbones, for example, polyamide (e.g., peptide nucleic acids (PNAs)) and polymorpholino (commercially available from the Anti-Virals, Inc., Corvallis, Oregon, as Neugene) polymers, and other synthetic sequence-specific nucleic acid polymers providing that the polymers contain nucleobases in a configuration which allows for base pairing and base stacking, such as is found in DNA and RNA. There is no intended distinction in length between the terms "polynucleotide," "oligonucleotide," "nucleic acid" and "nucleic acid molecule," and these terms will be used interchangeably. These terms refer only to the primary structure of the molecule. Thus, these terms include, for example, 3'-deoxy-2',5'-DNA, oligodeoxyribonucleotide N3' P5' phosphoramidates, 2'-O-alkyl-substituted RNA, double- and single-stranded DNA, as well as double- and single-stranded RNA, DNA:RNA hybrids, and hybrids between PNAs and DNA or RNA, and also include known types of modifications, for example, labels which are known in the art, methylation, "caps," substitution of one or more of the naturally occurring nucleotides with an analog, internucleotide modifications such as, for example, those with uncharged linkages (e.g., methyl phosphonates, phosphotriesters, phosphoramidates, carbamates, etc.), with negatively charged linkages (e.g., phosphorothioates, phosphorodithioates, etc.), and with positively charged linkages (e.g., aminoalklyphosphoramidates, aminoalkylphosphotriesters), those containing pendant moieties, such as, for example, proteins (including nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide. In particular, DNA is deoxyribonucleic acid.

As used herein, the term "target nucleic acid region" or "target nucleic acid" denotes a nucleic acid molecule with a "target sequence" to be amplified. The target nucleic acid may be either single-stranded or double-stranded and may include other sequences besides the target sequence, which may not be amplified. The term "target sequence" refers to the particular nucleotide sequence of the target nucleic acid which is to be amplified. The target sequence may include a probe-hybridizing region contained within the target molecule with which a probe will form a stable hybrid under desired conditions. The "target sequence" may also include the complexing sequences to which the oligonucleotide primers complex and be extended using the target sequence as a template. Where the target nucleic acid is originally single-stranded, the term "target sequence" also refers to the sequence complementary to the "target sequence" as present in the target nucleic acid. If the "target nucleic acid" is originally double-stranded, the term "target sequence" refers to both the plus (+) and minus (-) strands.

The term "primer" or "oligonucleotide primer" as used herein, refers to an oligonucleotide which acts to initiate synthesis of a complementary nucleic acid strand when placed under conditions in which synthesis of a primer extension product is induced, i.e., in the presence of nucleotides and a polymerization-inducing agent such as a DNA or RNA polymerase and at suitable temperature, pH, metal concentration, and salt concentration. The primer is preferably single-stranded for maximum efficiency in amplification, but may alternatively be double-stranded. If double-stranded, the primer is first treated to separate its strands before being used to prepare extension products. This denaturation step is typically effected by heat, but may alternatively be carried out using alkali, followed by neutralization. Thus, a "primer" is complementary to a template, and complexes by hydrogen bonding or hybridization with the template to give a primer/template complex for initiation of synthesis by a polymerase, which is extended by the addition of covalently bonded bases linked at its 3' end complementary to the template in the process of DNA synthesis.

As used herein, the term "probe" or "oligonucleotide probe" refers to a structure comprised of a polynucleotide, as defined above, that contains a nucleic acid sequence complementary to a nucleic acid sequence present in the target nucleic acid analyte. The polynucleotide regions of probes may be composed of DNA, and/or RNA, and/or synthetic nucleotide analogs. When an "oligonucleotide probe" is to be used in a 5' nuclease assay, such as the TaqMan^{™} technique, the probe will contain at least one fluorescer and at least one quencher which is digested by the 5' endonuclease activity of a polymerase used in the reaction in order to detect any amplified target oligonucleotide sequences. In this context, the oligonucleotide probe will have a sufficient number of phosphodiester linkages adjacent to its 5' end so that the 5' to 3' nuclease activity employed can efficiently degrade the bound probe to separate the fluorescers and quenchers. When an oligonucleotide probe is used in the TMA technique, it will be suitably labeled, as described below.

It will be appreciated that the hybridizing sequences need not have perfect complementarity to provide stable hybrids. In many situations, stable hybrids will form where fewer than about 10% of the bases are mismatches, ignoring loops of four or more nucleotides. Accordingly, as used herein the term "complementary" refers to an oligonucleotide that forms a stable duplex with its "complement" under assay conditions, generally where there is about 90% or greater homology.

The terms "hybridize" and "hybridization" refer to the formation of complexes between nucleotide sequences which are sufficiently complementary to form complexes via Watson-Crick base pairing. Where a primer "hybridizes" with target (template), such complexes (or hybrids) are sufficiently stable to serve the priming function required by, e.g., the DNA polymerase to initiate DNA synthesis.

As used herein, the term "binding pair" refers to first and second molecules that specifically bind to each other, such as complementary polynucleotide pairs capable of forming nucleic acid duplexes. "Specific binding" of the first member of the binding pair to the second member of the binding pair in a sample is evidenced by the binding of the first member to the second member, or vice versa, with greater affinity and specificity than to other components in the sample. The binding between the members of the binding pair is typically noncovalent. Unless the context clearly indicates otherwise, the terms "affinity molecule" and "target analyte" are used herein to refer to first and second members of a binding pair, respectively.

The terms "specific-binding molecule" and "affinity molecule" are used interchangeably herein and refer to a molecule that will selectively bind, through chemical or physical means to a detectable substance present in a sample. By "selectively bind" is meant that the molecule binds preferentially to the target of interest or binds with greater affinity to the target than to other molecules. For example, a nucleic acid molecule will bind to a substantially complementary sequence and not to unrelated sequences.

The "melting temperature" or "Tm" of double-stranded nucleic acid molecule is defined as the temperature at which half of the helical structure of the nucleic acid is lost due to heating or other dissociation of the hydrogen bonding between base pairs, for example, by acid or alkali treatment, or the like. The Tₘ of a nucleic acid molecule depends on its length and on its base composition. Nucleic acid molecules rich in GC base pairs have a higher Tₘ than those having an abundance of AT base pairs. Separated complementary strands of nucleic acids spontaneously reassociate or anneal to form duplex nucleic acids when the temperature is lowered below the Tₘ. The highest rate of nucleic acid hybridization occurs approximately 25°C below the Tₘ. The Tₘ may be estimated using the following relationship: Tₘ = 69.3 + 0.41 (GC)% (Marmur et al. (1962) J. Mol. Biol. 5:109-118).

As used herein, a "biological sample" refers to a sample of tissue or fluid isolated from a subject, that commonly includes antibodies produced by the subject. Typical samples include but are not limited to, blood, plasma, serum, fecal matter, urine, bone marrow, bile, spinal fluid, lymph fluid, samples of the skin, secretions of the skin, respiratory, intestinal, and genitourinary tracts, tears, saliva, milk, blood cells, organs, biopsies and also samples of *in vitro* cell culture constituents including but not limited to conditioned media resulting from the growth of cells and tissues in culture medium, e.g., recombinant cells, and cell components. Preferred biological samples are blood, plasma and serum.

As used herein, the terms "label" and "detectable label" refer to a molecule capable of detection, including, but not limited to, radioactive isotopes, fluorescers, chemiluminescers, chromophores, enzymes, enzyme substrates, enzyme cofactors, enzyme inhibitors, chromophores, dyes, metal ions, metal sols, ligands (e.g., biotin, avidin, strepavidin or haptens) and the like. The term "fluorescer" refers to a substance or a portion thereof which is capable of exhibiting fluorescence in the detectable range.

As used herein, a "solid support" refers to a solid surface such as a magnetic bead, latex bead, microtiter plate well, glass plate, nylon, agarose, acrylamide, and the like.

### II. Modes of Carrying out the Invention

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of compositions and methods similar or equivalent to those described herein can be used in the practice of the present invention, the preferred materials and methods are described herein.

As noted above, the present invention is based on the discovery of novel diagnostic methods for accurately detecting Hepatitis A virus (HAV) infection in a biological sample. The methods rely on sensitive nucleic acid-based detection techniques that allow identification of HAV target nucleic acid sequences in samples containing small amounts of virus. In particular, the inventor herein has discovered that the use of sequences from the 5' UTR of the HAV genome provides for rapid and sensitive detection of HAV in biological samples. The sequences for the HAV genome, including the 5' UTR, in a number of HAV isolates are known. See, for example, NCBI accession numbers K02990; AB020564; AB020565; AB020566; AB020567; AB020568; AB020569; AF268396; M16632; M14707; M20273; NC001489; X83302; Cohen et al. J. Virol. (1987) 61:50-59. By comparing the sequences from the various HAV isolates, these and other 5' UTR sequences for use with the present invention can be readily identified. For convenience, the various nucleic acid molecules for use with the present invention have been numbered relative to NCBI Accession No. K02990. The 5' UTR sequence occurs at positions 1-723 of NCBI Accession No. K02990.

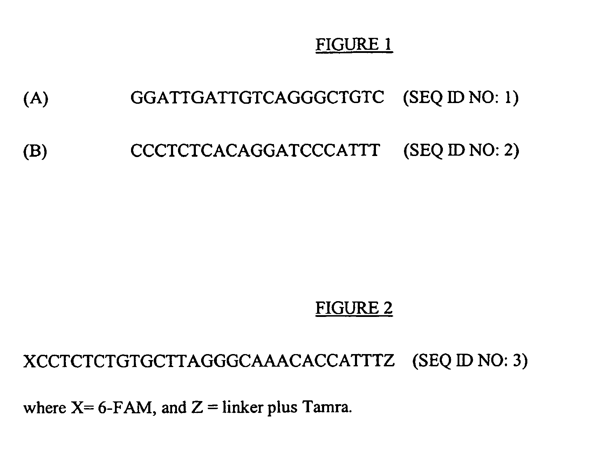

In the strategy of the present invention, the target nucleic acids are separated from non-homologous DNA/RNA. The target nucleic acids are separated by using capture oligonucleotides immobilized on a solid support, where the capture oligonucleotides can be specific for the organism to be detected. For HAV, the separated target nucleic acids are amplified using the primers in the 5' UTR. Primers from this region are primers comprising the sequence of SEQ ID NOS:1 and 2 (Figure 1).

In one aspect of the present invention the biological sample potentially carrying target nucleic acid is contacted with a solid support having capture oligonucleotides. The capture oligonucleotides may be associated with the solid support, for example, by covalent binding of the probe moiety to the solid support, by affinity association, hydrogen binding, or nonspecific association.

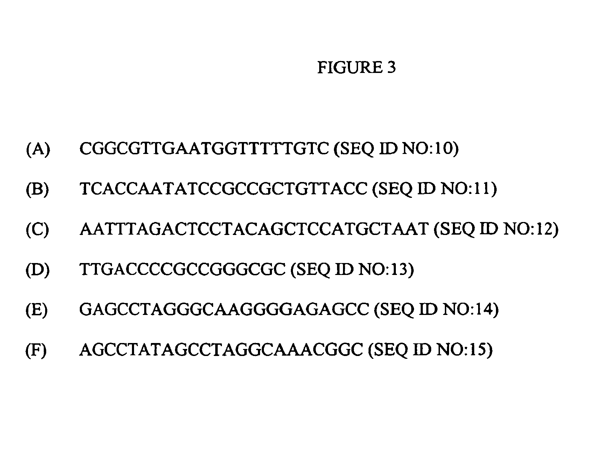

The capture oligonucleotides are 10 to about 60 nucleotides, or any integer within these ranges, such as a sequence including 18, 19, 20, 21, 22, 23, 24, 25, 26...35...40, etc. nucleotides from the region of interest. Capture oligonucleotides are derived from the 5' UTR sequence of an HAV isolate, such as those depicted in Figures 3A-3F (SEQ ID NOS:10-15) herein.

The capture oligonucleotide may be attached to the solid support in a variety of manners. For example, the oligonucleotide may be attached to the solid support by attachment of the 3' or 5' terminal nucleotide of the capture oligonucleotide to the solid support. More preferably, the capture oligonucleotide is attached to the solid support by a linker which serves to distance the probe from the solid support. The linker is usually at least 10-50 atoms in length, more preferably at least 15-30 atoms in length. The required length of the linker will depend on the particular solid support used. For example, a six atom linker is generally sufficient when high cross-linked polystyrene is used as the solid support.

A wide variety of linkers are known in the art which may be used to attach the capture oligonucleotide to the solid support. The linker may be formed of any compound which does not significantly interfere with the hybridization of the target sequence to the capture oligonucleotide attached to the solid support. The linker may be formed of a homopolymeric oligonucleotide which can be readily added on to the linker by automated synthesis. The homopolymeric sequence can be either 5' or 3' to the virus-specific sequence. In one aspect of the invention, the capture oligonucleotides can be linked to a homopolymer chain, such as, for example poly A, poly T, poly G, poly C, poly U, poly dA, poly dT, poly dG, poly dC, or poly dU in order to facilitate attachment to the solid support. The homopolymer chain can be from about 10 to about 40 nucleotides in length, or preferably about 12 to about 25 nucleotides in length, or any integer within these ranges, such as for example, 10...12...16, 17, 18, 19, 20, 21, 22, 23, or 24 nucleotides.

Representative homopolymeric sequences include poly T or poly A sequences. Alternatively, polymers such as functionalized polyethylene glycol can be used as the linker. Such polymers do not significantly interfere with the hybridization of probe to the target oligonucleotide. Examples of linkages include polyethylene glycol, carbamate and amide linkages. The linkages between the solid support, the linker and the capture oligonucleotide are preferably not cleaved during removal of base protecting groups under basic conditions at high temperature.

The capture oligonucleotide may also be phosphorylated at the 3' end in order to prevent extension of the capture oligonucleotide.

The solid support disclosed herein may take many forms including, for example, nitrocellulose reduced to particulate form and retrievable upon passing the sample medium containing the support through a sieve; nitrocellulose or the materials impregnated with magnetic particles or the like, allowing the nitrocellulose to migrate within the sample medium upon the application of a magnetic field; beads or particles which may be filtered or exhibit electromagnetic properties; polystyrene beads which partition to the surface of an aqueous medium; and magnetize silica. Examples of preferred types of solid supports for immobilization of the capture oligonucleotide include controlled pore glass, glass plates, polystyrene, avidin- coated polystyrene beads, cellulose, nylon, acrylamide gel and activated dextran.

One aspect of the present invention includes a solid support comprising magnetic beads, optionally the magnetic beads contain primary amine functional groups which facilitate covalent binding or association of the capture oligonucleotides to the magnetic support particles. Alternatively, the magnetic beads have immobilized thereon homopolymers, such as poly T or poly A sequences. The use of a solid support with magnetic beads allows for a one-pot method of isolation, amplification and detection as the solid support can be separated from the biological sample by magnetic means.

The magnetic beads can be produced using standard techniques or obtained from commercial sources. In general, the beads may be comprised of magnetic particles, although they can also be other magnetic metal or metal oxides, whether in impure, alloy, or composite form, as long as they have a reactive surface and exhibit an ability to react to a magnetic field. Other materials that may be used individually or in combination with iron include, but are not limited to, cobalt, nickel, and silicon. A magnetic bead suitable for the application in the present invention includes magnetic beads containing poly dT groups marketed under the trade name Sera-Mag^{™} magnetic oligonucleotide beads by Seradyn, Indianopolis, IN. Magnetic silica suitable for the application in the present invention includes MagPrep^{™} magnetic silica by Novagen, Madison, WI.

Next, the association of the capture oligonucleotides with the solid support is initiated by contacting the solid support with the medium containing the capture oligonucleotides. In one aspect, the magnetic bead containing poly dT groups is hybridized with the target sequences that comprise poly dA contiguous with the sequence selected from the conserved single stranded region of the HAV genome. The poly dA on the capture oligonucleotide and the poly dT on the solid support hybridize thereby immobilizing or associating the capture oligonucleotides with the solid support. In another aspect, the magnetic bead has immobilized on its surface nucleotide sequences of about 10 to about 75 nucleotides, preferably about 10 to about 25 nucleotides derived from the nucleotide sequences disclosed in WO 03/031934.

The solid support is brought into contact with the biological sample under high concentrations of chaotropic salts or under hybridizing conditions. The capture oligonucleotides hybridize to the target strands present in the biological sample. Typically, hybridizations of capture oligonucleotides to the targets can be accomplished in approximately 15 minutes, but may take as long as 3 to 48 hours.

In another aspect of the disclosure, silica magnetic particles are exposed to the medium containing the target material under conditions designed to promote the formation of a complex. The complex is more preferably formed in a mixture of the silica magnetic particle, the medium, and a chaotropic salt.

Chaotropic salts are salts of chaotropic ions that are highly soluble in aqueous solutions. The chaotropic ions provided by such salts, at sufficiently high concentration in aqueous solutions of proteins or nucleic acids, cause proteins to unfold, nucleic acids to lose secondary structure or, in the case of double-stranded nucleic acids, melt. It is thought that chaotropic ions have these effects because they disrupt hydrogen-bonding networks that exists in liquid water and thereby make denatured proteins and nucleic acids thermodynamically more stable than their correctly folded or structured counterparts. Representative chaotropic ions include, but are not limited to, guanidinium, iodide, perchlorate and trichloroacetate. Preferred is the guanidinium ion. Chaotropic salts include, but are not limited to, guanidine hydrochloride, guanidine thiocyanate, sodium iodide, sodium perchlorate, and sodium trichloroacetate. Preferred are the guanidinium salts, and particularly preferred is guanidine thiocyanate.

The concentration of chaotropic ions for use in this practice is preferably between about 0.1 M and 7 M, but more preferably between about 0.5 M and 5 M. The concentration of chaotropic ions in the mixture must be sufficiently high to cause the biological target material to adhere to the silica magnetic particles in the mixture, but not so high as to substantially denature, to degrade, or to cause the target material to precipitate out of the mixture. Proteins and large molecules of double-stranded nucleic acid, such as viral nucleic acids, are stable at chaotropic salt concentrations between 0.5 and 2 M, but are known to precipitate out of solution at chaotropic salt concentrations above about 2 M.

In one aspect, the complex formed as described above is incubated until at least some of the nucleic acid material is adhered to the silica magnetic particle to form a complex. This incubation step is carried out at a temperature of at least about 0 °C, preferably at least about 4 °C, and more preferably at least about 20 °C, provided that the incubation temperature is not more than about 75 °C. Thus, temperatures in the ranges of 0 °C to 75 °C, preferably 4 °C to 50 °C, and most preferably, about 15 °C to about 35 °C, or any integer within these ranges will find use herein. The incubation step is preferably carried out at a temperature below the temperature at which the silica magnetic particles begin to loose their capacity to reversibly bind the nucleic acid material, and may be carried out at about room temperature (i.e. at about 25 °C).

The solid support is then separated from the biological sample by filtering, passing through a column, or by magnetic means. As will be appreciated by one of skill in the art, the method of separation will depend on the type of solid support selected. Since the targets are hybridized to the capture oligonucleotides immobilized on the solid support, the target strands are thereby separated from the impurities in the sample. In some cases, extraneous nucleic acids, proteins, carbohydrates, lipids, cellular debris, and other impurities may still be bound to the support, although at much lower concentrations than initially found in the biological sample. Those skilled in the art will recognize that some undesirable materials can be removed by washing the support with a washing medium. The separation of the solid support from the biological sample preferably removes at least about 70%, more preferably about 90% and, most preferably, at least about 95% of the non-target nucleic acids present in the sample.

The methods of the present invention also include amplifying the captured target oligonucleotide to produce amplified nucleic acids. Amplifying a target nucleic acid uses a nucleic acid polymerase to produce multiple copies of the target oligonucleotide or fragments thereof. Suitable amplification techniques are well known in the art, such as, for example transcription associated amplification, polymerase chain reaction (PCR), replicase mediated amplification, and ligase chain reaction (LCR).

The primers for use with the assays of the invention are preferably unique for the organism the presence of which is to be detected. Thus, for the detection of HAV, the primers are derived from the conserved regions in the untranslated region of HAV, such as those shown in Figure 1.

Primers and capture oligonucleotides for use in the assays are readily synthesized by standard techniques, e.g., solid phase synthesis via phosphoramidite chemistry, as disclosed in U.S. Patent Nos. 4,458,066 and 4,415,732; Beaucage et al. (1992) Tetrahedron 48:2223-2311; and Applied Biosystems User Bulletin No. 13 (1 April 1987). Other chemical synthesis methods include, for example, the phosphotriester method described by Narang et al., Meth. Enzymol. (1979) 68:90 and the phosphodiester method disclosed by Brown et al., Meth. Enzymol. (1979) 68:109. Poly(A) or poly(C), or other non-complementary nucleotide extensions may be incorporated into probes using these same methods. Hexaethylene oxide extensions may be coupled to probes by methods known in the art. Cload et al. (1991) J. Am. Chem. Soc. 113:6324-6326; U.S. Patent No. 4,914,210 to Levenson et al.; Durand et al. (1990) Nucleic Acids Res. 18:6353-6359; and Horn et al. (1986) Tet. Lett. 27:4705-4708. The primer sequences are in the range of between 10-60 nucleotides in length, such as 15-60, 20-40 and so on, more typically in the range of between 18-40 nucleotides long, and any length between the stated ranges. The typical probe is in the range of between 10-50 nucleotides long, such as 15-40, 18-30, and so on, and any length between the stated ranges.

Moreover, the probes may be coupled to labels for detection. There are several means known for derivatizing oligonucleotides with reactive functionalities which permit the addition of a label. For example, several approaches are available for biotinylating probes so that radioactive, fluorescent, chemiluminescent, enzymatic, or electron dense labels can be attached via avidin. See, e.g., Broken et al., Nucl. Acids Res. (1978) 5:363-384 which discloses the use of ferritin-avidin-biotin labels; and Chollet et al. Nucl. Acids Res. (1985) 13:1529-1541 which discloses biotinylation of the 5' termini of oligonucleotides via an aminoalkylphosphoramide linker arm. Several methods are also available for synthesizing amino-derivatized oligonucleotides which are readily labeled by fluorescent or other types of compounds derivatized by amino-reactive groups, such as isothiocyanate, N-hydroxysuccinimide, or the like, see, e.g., Connolly (1987) Nucl. Acids Res. 15:3131-3139, Gibson et al. (1987) Nucl. Acids Res. 15:6455-6467 and U.S. Patent No. 4,605,735 to Miyoshi et al. Methods are also available for synthesizing sulfhydryl-derivatized oligonucleotides which can be reacted with thiol-specific labels, see, e.g., U.S. Patent No. 4,757,141 to Fung et al., Connolly et al. (1985) Nucl. Acids Res. 13:4485-4502 and Spoat et al. (1987) Nucl. Acids Res. 15:4837-4848. A comprehensive review of methodologies for labeling nucleic acid fragments is provided in Matthews et al., Anal. Biochem. (1988) 169:1-25.

For example, probes may be fluorescently labeled by linking a fluorescent molecule to the non-ligating terminus of the probe. Guidance for selecting appropriate fluorescent labels can be found in Smith et al., Meth. Enzymol. (1987) 155:260-301; Karger et al., Nucl. Acids Res. (1991) 19:4955-4962; Haugland (1989) Handbook of Fluorescent Probes and Research Chemicals (Molecular Probes, Inc., Eugene, OR). Preferred fluorescent labels include fluorescein and derivatives thereof, such as disclosed in U.S. Patent No. 4,318,846 and Lee et al., Cytometry (1989) 10:151-164, and 6-FAM (fluorescein), JOE (2',7'-dimethoxy-4',5'-dichlorofluorescein), TAMRA (tetramethylrhodamine), ROX (rhodaniine X), HEX-1, HEX-2, ZOE, TET-1 or NAN-2, and the like.

Additionally, probes can be labeled with an acridinium ester (AE) using the techniques described below. Current technologies allow the AE label to be placed at any location within the probe. See, e.g., Nelson et al. (1995) "Detection of Acridinium Esters by Chemiluminescence" in Nonisotopic Probing, Blotting and Sequencing, Kricka L.J.(ed) Academic Press, San Diego, CA; Nelson et al. (1994) "Application of the Hybridization Protection Assay (HPA) to PCR" in The Polymerase Chain Reaction, Mullis et al. (eds.) Birkhauser, Boston, MA; Weeks et al., Clin. Chem. (1983) 29:1474-1479; Berry et al., Clin. Chem. (1988) 34:2087-2090. An AE molecule can be directly attached to the probe using non-nucleotide-based linker arm chemistry that allows placement of the label at any location within the probe. See, e.g., U.S. Patent Nos. 5,585,481 and 5,185,439.

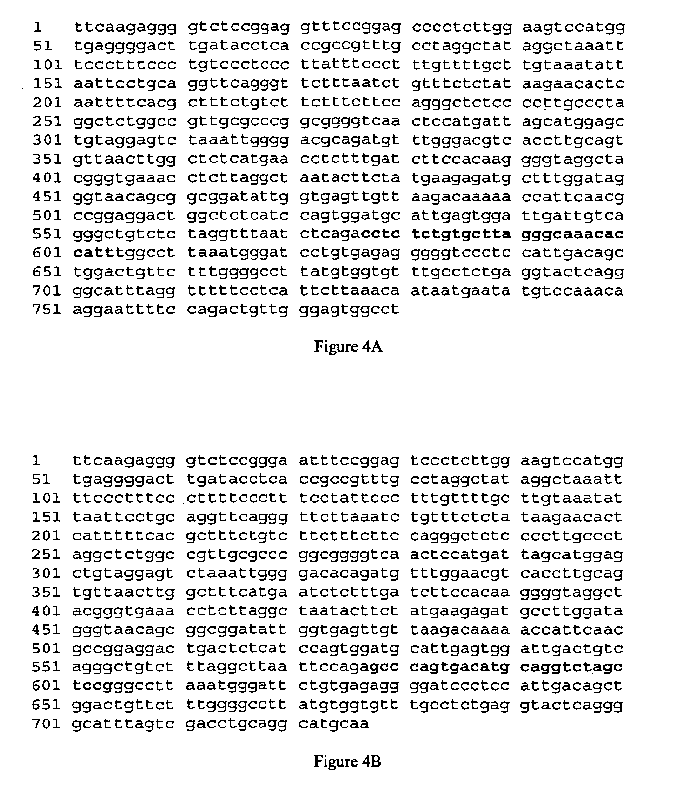

In certain embodiments, an internal control (IC) or an internal standard is added to serve as a control for target capture and amplification. Preferably, the IC includes a sequence that differs from the target sequences, is capable of hybridizing with the probe sequences used for separating the oligonucleotides specific for the organism from the sample, and is capable of amplification. The use of the internal control permits the control of the separation process, the amplification process, and the detection system, and permits the monitoring of the assay performance and quantization for the sample(s). The IC can be included at any suitable point, for example, in the lysis buffer. In one embodiment, the IC comprises RNA containing a part of the HAV nucleotide sequence and a unique sequence that hybridizes with the probe. Thus, in certain embodiments, the IC includes a portion of the HAV genome with a modified sequence with 5-30, such as 6...9...12...15...20 and so on or more bases substituted with other bases. The substitute bases can be located over the entire length of the target sequence such that only 2 or 3 consecutive sequences are replaced. A representative IC for HAV is shown in Figure 4B and comprises 721 bps derived from the 5' UTR of the HAV genome. The bolded, upper case bases in Figure 4B represent bases that have been substituted for the bases occurring in the wild-type sequence (see Figure 4A). The assay may additionally include probes specific to the internal standard (IC probe).

Representative probes for the IC sequence are detailed in the examples as SEQ ID NOS:18 and 19. The IC probe can optionally be coupled with a detectable label that is different from the detectable label for the target sequence. In embodiments where the detectable label is a fluorophore, the IC can be quantified spectrophotometrically and by limit of detection studies.

Typically, the copy number of IC which does not interfere with the target detection is determined by titrating the IC with a fixed IU/copies/PFU of target, preferably at the lower end, and a standard curve is generated by diluting a sample of internationally accepted standard.

In another embodiment, an IC, as described herein, is combined with RNA isolated from the sample according to standard techniques known to those of skill in the art. The RNA is then reverse transcribed using a reverse transcriptase to provide cDNA. The cDNA sequences can be optionally amplified (e.g., by PCR) using labeled primers. The amplification products are separated, typically by electrophoresis, and the amount of incorporated label (proportional to the amount of amplified product) is determined. The amount of mRNA in the sample is then calculated by comparison with the signal produced by the known standards.

The primers and probes described above may be used in polymerase chain reaction (PCR)-based techniques to detect HAV infection in biological samples. PCR is a technique for amplifying a desired target nucleic acid sequence contained in a nucleic acid molecule or mixture of molecules. In PCR, a pair of primers is employed in excess to hybridize to the complementary strands of the target nucleic acid. The primers are each extended by a polymerase using the target nucleic acid as a template. The extension products become target sequences themselves after dissociation from the original target strand. New primers are then hybridized and extended by a polymerase, and the cycle is repeated to geometrically increase the number of target sequence molecules. The PCR method for amplifying target nucleic acid sequences in a sample is well known in the art and has been described in, e.g., Innis et al. (eds.) PCR Protocols (Academic Press, NY 1990); Taylor (1991) Polymerase chain reaction: basic principles and automation, in PCR: A Practical Approach, McPherson et al. (eds.) IRL Press, Oxford; Saiki et al. (1986) Nature 324:163; as well as in U.S. Patent Nos. 4,683,195, 4,683,202 and 4,889,818.

In particular, PCR uses relatively short oligonucleotide primers which flank the target nucleotide sequence to be amplified, oriented such that their 3' ends face each other, each primer extending toward the other. The polynucleotide sample is extracted and denatured, prefer-ably by heat, and hybridized with first and second primers which are present in molar excess. Polymerization is catalyzed in the presence of the four deoxyribonucleotide triphosphates (dNTPs -- dATP, dGTP, dCTP and dTTP) using a primer- and template-dependent polynucleotide polymerizing agent, such as any enzyme capable of producing primer extension products, for example, *E. coli* DNA polymerase I, Klenow fragment of DNA polymerase I, T4 DNA polymerase, thermostable DNA polymerases isolated from *Thermus aquaticus* (*Taq*), available from a variety of sources (for example, Perkin Elmer), *Thermus thermophilus* (United States Biochemicals), *Bacillus stereothermophilus* (Bio-Rad), or *Thermococcus litoralis* ("Vent" polymerase, New England Biolabs). This results in two "long products" which contain the respective primers at their 5' ends covalently linked to the newly synthesized complements of the original strands. The reaction mixture is then returned to polymerizing conditions, e.g., by lowering the temperature, inactivating a denaturing agent, or adding more polymerase, and a second cycle is initiated. The second cycle provides the two original strands, the two long products from the first cycle, two new long products replicated from the original strands, and two "short products" replicated from the long products. The short products have the sequence of the target sequence with a primer at each end. On each additional cycle, an additional two long products are produced, and a number of short products equal to the number of long and short products remaining at the end of the previous cycle. Thus, the number of short products containing the target sequence grow exponentially with each cycle. Preferably, PCR is carried out with a commercially available thermal cycler, e.g., Perkin Elmer.

RNAs may be amplified by reverse transcribing the mRNA into cDNA, and then performing PCR (RT-PCR), as described above. Alternatively, a single enzyme may be used for both steps as described in U.S. Patent No. 5,322,770. mRNA may also be reverse transcribed into cDNA, followed by asymmetric gap ligase chain reaction (RT-AGLCR) as described by Marshall et al. (1994) PCR Meth. App. 4:80-84.

The fluorogenic 5' nuclease assay, known as the TaqMan^{™} assay (Perkin-Elmer), is a powerful and versatile PCR-based detection system for nucleic acid targets. Hence, primers and probes derived from regions of the HAV genome described herein can be used in TaqMan^{™} analyses to detect the presence of infection in a biological sample. Analysis is performed in conjunction with thermal cycling by monitoring the generation of fluorescence signals. The assay system dispenses with the need for gel electrophoretic analysis, and has the capability to generate quantitative data allowing the determination of target copy numbers.

The fluorogenic 5' nuclease assay is conveniently performed using, for example, AmpliTaq Gold^{™} DNA polymerase, which has endogenous 5' nuclease activity, to digest an internal oligonucleotide probe labeled with both a fluorescent reporter dye and a quencher (see, Holland et al., Proc. Natl. Acad.Sci. USA (1991) 88:7276-7280; and Lee et al., Nucl. Acids Res. (1993) 21:3761-3766). Assay results are detected by measuring changes in fluorescence that occur during the amplification cycle as the fluorescent probe is digested, uncoupling the dye and quencher labels and causing an increase in the fluorescent signal that is proportional to the amplification of target nucleic acid.

The amplification products can be detected in solution or using solid supports. In this method, the TaqMan^{™} probe is designed to hybridize to a target sequence within the desired PCR product. The 5' end of the TaqMan^{™} probe contains a fluorescent reporter dye. The 3' end of the probe is blocked to prevent probe extension and contains a dye that will quench the fluorescence of the 5' fluorophore. During subsequent amplification, the 5' fluorescent label is cleaved off if a polymerase with 5'exonuclease activity is present in the reaction. Excision of the 5' fluorophore results in an increase in fluorescence which can be detected.

Accordingly, in one aspect, the present invention relates to methods for amplifying a target HAV nucleotide sequence using a nucleic acid polymerase having 5' to 3' nuclease activity, one or more primers capable of hybridizing to the HAV target sequence, and an oligonucleotide probe capable of hybridizing to the HAV target sequence 3' relative to the primer. During amplification, the polymerase digests the oligonucleotide probe when it is hybridized to the target sequence, thereby separating the reporter molecule from the quencher molecule. As the amplification is conducted, the fluorescence of the reporter molecule is monitored, with fluorescence corresponding to the occurrence of nucleic acid amplification. The reporter molecule is preferably a fluorescein dye and the quencher molecule is preferably a rhodamine dye.

While the length of the primers and probes can vary, the probe sequences are selected such that they have a higher melt temperature than the primer sequences. Preferably, the probe sequences have an estimated melt temperature that is about 10 °C higher than the melt temperature for the amplification primer sequences. Hence, the primer sequences are generally shorter than the probe sequences. Typically, the primer sequences are in the range of between 10-75 nucleotides long, more typically in the range of 20-45. The typical probe is in the range of between 10-50 nucleotides long, more typically 15-40 nucleotides in length.

For a detailed description of the TaqMan^{™} assay, reagents and conditions for use therein, see, e.g., Holland et al., Proc. Natl. Acad. Sci, U.S.A. (1991) 88:7276-7280; U.S. Patent Nos. 5,538,848, 5,723,591, and 5,876,930.

The HAV sequences described herein may also be used as a basis for transcription-mediated amplification (TMA) assays. TMA provides a method of identifying target nucleic acid sequences present in very small amounts in a biological sample. Such sequences may be difficult or impossible to detect using direct assay methods. In particular, TMA is an isothemal, autocatalytic nucleic acid target amplification system that can provide more than a billion RNA copies of a target sequence. The assay can be done qualitatively, to accurately detect the presence or absence of the target sequence in a biological sample. The assay can also provide a quantitative measure of the amount of target sequence over a concentration range of several orders of magnitude. TMA provides a method for autocatalytically synthesizing multiple copies of a target nucleic acid sequence without repetitive manipulation of reaction conditions such as temperature, ionic strength and pH.

Generally, TMA includes the following steps: (a) isolating nucleic acid, including RNA, from the biological sample of interest suspected of being infected with HAV; and (b) combining into a reaction mixture (i) the isolated nucleic acid, (ii) first and second oligonucleotide primers, the first primer having a complexing sequence sufficiently complementary to the 3' terminal portion of an RNA target sequence, if present (for example the (+) strand), to complex therewith, and the second primer having a complexing sequence sufficiently complementary to the 3' terminal portion of the target sequence of its complement (for example, the (-) strand) to complex therewith, wherein the first oligonucleotide further comprises a sequence 5' to the complexing sequence which includes a promoter, (iii) a reverse transcriptase or RNA and DNA dependent DNA polymerases, (iv) an enzyme activity which selectively degrades the RNA strand of an RNA-DNA complex (such as an RNAse H) and (v) an RNA polymerase which recognizes the promoter.

The components of the reaction mixture may be combined stepwise or at once. The reaction mixture is incubated under conditions whereby an oligonucleotide/target sequence is formed, including nucleic acid priming and nucleic acid synthesizing conditions (including ribonucleotide triphosphates and deoxyribonucleotide triphosphates) for a period of time sufficient to provide multiple copies of the target sequence. The reaction advantageously takes place under conditions suitable for maintaining the stability of reaction components such as the component enzymes and without requiring modification or manipulation of reaction conditions during the course of the amplification reaction. Accordingly, the reaction may take place under conditions that are substantially isothermal and include substantially constant ionic strength and pH. The reaction conveniently does not require a denaturation step to separate the RNA-DNA complex produced by the first DNA extension reaction.

Suitable DNA polymerases include reverse transcriptases, such as avian myeloblastosis virus (AMV) reverse transcriptase (available from, e.g., Seikagaku America, Inc.) and Moloney murine leukemia virus (MMLV) reverse transcriptase (available from, e.g., Bethesda Research Laboratories).

Promoters or promoter sequences suitable for incorporation in the primers are nucleic acid sequences (either naturally occurring, produced synthetically or a product of a restriction digest) that are specifically recognized by an RNA polymerase that recognizes and binds to that sequence and initiates the process of transcription whereby RNA transcripts are produced. The sequence may optionally include nucleotide bases extending beyond the actual recognition site for the RNA polymerase which may impart added stability or susceptibility to degradation processes or increased transcription efficiency. Examples of useful promoters include those which are recognized by certain bacteriophage polymerases such as those from bacteriophage T3, T7 or SP6, or a promoter from *E*. *coli.* These RNA polymerases are readily available from commercial sources, such as New England Biolabs and Epicentre.

Some of the reverse transcriptases suitable for use in the methods herein have an RNAse H activity, such as AMV reverse transcriptase. It may, however, be preferable to add exogenous RNAse H, such as *E*. *coli* RNAse H, even when AMV reverse transcriptase is used. RNAse H is readily available from, e.g., Bethesda Research Laboratories.

The RNA transcripts produced by these methods may serve as templates to produce additional copies of the target sequence through the above-described mechanisms. The system is autocatalytic and amplification occurs autocatalytically without the need for repeatedly modifying or changing reaction conditions such as temperature, pH, ionic strength or the like.

Detection may be done using a wide variety of methods, including direct sequencing, hybridization with sequence-specific oligomers, gel electrophoresis and mass spectrometry these methods can use heterogeneous or homogeneous formats, isotopic or nonisotopic labels, as well as no labels at all.

One preferable method of detection is the use of target sequence-specific oligonucleotide probes described above. The probes may be used in hybridization protection assays (HPA). In this embodiment, the probes are conveniently labeled with acridinium ester (AE), a highly chemiluminescent molecule. See, e.g., Nelson et al. (1995) "Detection of Acridinium Esters by Chemiluminescence" in Nonisotopic Probing, Blotting and Sequencing, Kricka L.J.(ed) Academic Press, San Diego, CA; Nelson et al. (1994) "Application of the Hybridization Protection Assay (HPA) to PCR" in The Polymerase Chain Reaction, Mullis et al. (eds.) Birkhauser, Boston, MA; Weeks et al., Clin. Chem. (1983) 29:1474-1479; Berry et al., Clin. Chem. (1988) 34:2087-2090. One AE molecule is directly attached to the probe using a non-nucleotide-based linker arm chemistry that allows placement of the label at any location within the probe. See, e.g., U.S. Patent Nos. 5,585,481 and 5,185,439. Chemiluminescence is triggered by reaction with alkaline hydrogen peroxide which yields an excited N-methyl acridone that subsequently collapses to ground state with the emission of a photon.

When the AE molecule is covalently attached to a nucleic acid probe, hydrolysis is rapid under mildly alkaline conditions. When the AE-labeled probe is exactly complementary to the target nucleic acid, the rate of AE hydrolysis is greatly reduced. Thus, hybridized and unhybridized AE-labeled probe can be detected directly in solution, without the need for physical separation.

HPA generally consists of the following steps: (a) the AE-labeled probe is hybridized with the target nucleic acid in solution for about 15 to about 30 minutes. A mild alkaline solution is then added and AE coupled to the unhybridized probe is hydrolyzed. This reaction takes approximately 5 to 10 minutes. The remaining hybrid-associated AE is detected as a measure of the amount of target present. This step takes approximately 2 to 5 seconds. Preferably, the differential hydrolysis step is conducted at the same temperature as the hybridization step, typically at 50 to 70 °C. Alternatively, a second differential hydrolysis step may be conducted at room temperature. This allows elevated pHs to be used, for example in the range of 10-11, which yields larger differences in the rate of hydrolysis between hybridized and unhybridized AE-labeled probe. HPA is described in detail in, e.g., U.S. Patent Nos. 6,004,745; 5,948,899; and 5,283,174.

TMA is described in detail in, e.g., U.S. Patent No. 5,399,491. In one example of a typical assay, an isolated nucleic acid sample, suspected of containing a HAV target sequence, is mixed with a buffer concentrate containing the buffer, salts, magnesium, nucleotide triphosphates, primers, dithiothreitol, and spermidine. The reaction is optionally incubated at about 100 °C for approximately two minutes to denature any secondary structure. After cooling to room temperature, reverse transcriptase, RNA polymerase, and RNAse H are added and the mixture is incubated for two to four hours at 37 °C. The reaction can then be assayed by denaturing the product, adding a probe solution, incubating 20 minutes at 60 °C, adding a solution to selectively hydrolyze the unhybridized probe, incubating the reaction six minutes at 60 °C, and measuring the remaining chemiluminescence in a luminometer.

As is readily apparent, design of the assays described herein are subject to a great deal of variation, and many formats are known in the art. The above descriptions are merely provided as guidance and one of skill in the art can readily modify the described protocols, using techniques well known in the art.

The above-described assay reagents, including the primers, probes, solid support with bound probes, as well as other detection reagents, can be provided in kits, with suitable instructions and other necessary reagents, in order to conduct the assays as described above. The kit will normally contain in separate containers the combination of primers and probes (either already bound to a solid matrix or separate with reagents for binding them to the matrix), control formulations (positive and/or negative), labeled reagents when the assay format requires same and signal generating reagents (e.g., enzyme substrate) if the label does not generate a signal directly. Instructions (e.g., written, tape, VCR, CD-ROM, etc.) for carrying out the assay usually will be included in the kit. The kit can also contain, depending on the particular assay used, other packaged reagents and materials (i.e. wash buffers and the like). Standard assays, such as those described above, can be conducted using these kits.

### III. Experimental

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

In the following examples, enzymes were purchased from commercial sources, and used according to the manufacturers' directions. Nitrocellulose filters and the like were also purchased from commercial sources.

In the isolation of RNA and DNA fragments, except where noted, all RNA and DNA manipulations were done according to standard procedures. See, Sambrook et al., *supra.* Restriction enzymes, T₄ DNA ligase, *E*. *coli,* DNA polymerase I, Klenow fragment, and other biological reagents can be purchased from commercial suppliers and used according to the manufacturers' directions. Double stranded nucleic acid fragments were separated on agarose gels.

### Example 1

### Extraction of HAV RNA from the Biological Sample

HAV nucleic acid-positive serum was purchased from BioClinical Partners (Berkeley, CA). Two approaches were used to isolate nucleic acid from sample. In particular, RNA was extracted by (a) binding to silica; and (b) annealing to target-specific oligonucleotides.

### (a) Isolation of nucleic acid by binding to silica.

The RNA was extracted by binding to silica using the method described by Boom, R. et al. (1990) "Rapid and simple method for purification of nucleic acids" J. Clin. Microbiol. 28, 495-503. In the presence of high concentrations of chaotropic salt such as guanidinium isothiocyanate, nucleic acids bind to silica. Small sized nucleic acids bind more efficiently to silica under conditions of acidic pH. The bound nucleic acids are efficiently eluted in low salt, alkaline pH buffer at high temperatures. The substitution of magnetized silica for regular silica greatly facilitates washing and elution steps of nucleic acid isolation. A magnetic base was used to capture the nucleic acid-bound silica particles, thus eliminating centrifugations required to sediment regular silica particles.

The lysis buffer used was from Organon-Teknika (Durham, NC). This lysis buffer contains guanidinium isothiocyanate to solubilize proteins and inactivate RNases and DNases. The detergent Triton X-100 further facilitates the process of solubilization and disintegration of cell structure and nuclear proteins, thus releasing nucleic acid. The lysis reagent was acidified to enhance nucleic acid binding, and 50 µl of alkaline elution buffer was used to elute the bound nucleic acid. The pre-aliquotted 9.0 ml lysis reagent was used to extract nucleic acid form 2.0 ml of HAV IgM positive plasma. Magnetized silica (MagPrep particles from Novagen, Madison, WI) was used to capture the nucleic acid-bound silica particles, thus eliminating centrifugations required to sediment regular silica particles. The bound nucleic acids were eluted in 50 µl of 10 mM Tris pH 9.0 containing 1 mM EDTA. Following nucleic acid isolation, the presence of HAV was determined by performing TaqMan^{™} PCR, as described below.

### (b) Isolation of nucleic acid by annealing to target-specific oligonucleotides.

Although use of magnetized silica greatly facilitates rapid and easy handling during the washing and elution steps, isolation of nucleic acid is still laborious and time consuming. Therefore one-step capture of specific nucleic acid target from plasma or serum using magnetic beads was used. In order to make this applicable for a wide variety of viral nucleic acid capture tests, generic magnetic beads coupled with oligo dT were used. Sera-Mag magnetic oligo (dT) beads (Seradyn, Indianapolis, IN) with an oligo dT length of about 14 bps, were used in combination with Capture oligonucleotides containing a poly A tail at the 3' end contiguous with the HAV-specific sequence (designated at the end of the sequence specified below).

The magnetic beads were suspended in 0.4 ml of primer-less TMA lysis buffer (GenProbe, San Diego, CA) and the capture primers were tested individually or in combination. Following capture, the beads were washed three times with a wash buffer of 10 mM Hepes (pH 7.5), 0.5% NP-40 containing 0.3 M NaCl. The beads with the captured nucleic acid were suspended in 100 µl of TaqMan^{™} one-step RT-PCR reagent and transferred to a TaqMan^{™} RT-PCR microtiter plate for detection by TaqMan^{™} PCR as described below. Several oligonucleotide combinations were efficient at capturing HAV as detected by the TaqMan^{™} assay.

The capture oligonucleotides used were as follows (the numbering indicated at the end of the sequence corresponds to the position within the HAV genome, relative to NCBI accession number K02990. The capture sequences are reverse complementary sequences to the specified positions, since HAV is a positive strand RNA virus.):
CGGCGTTGAATGGTTTTTGTCAAAAAAAAAAAAAAAAAAAAAAp
   (nt483-503, plus a 22 bp polyA tail, p=phosphorylated ) (SEQ ID NO:4)
TCACCAATATCCGCCGCTGTTACCAAAAAAAAAAAAAAAAAAAAAAp
   (nt451-474, plus a 22 bp polyA tail, p=phosphorylated) (SEQ ID NO:5)
AATTTAGACTCCTACAGCTCCATGCTAATAAAAAAAAAAAAAAAAAAAAAAp
   (nt291-319, plus a 22 bp polyA tail, p=phosphorylated) (SEQ ID NO:6)
TTGACCCCGCCGGGCGCAAAAAAAAAAAAAAAAAAAAAAp
   (264-280, plus a 22 bp polyA tail, p=phosphorylated) (SEQ ID NO:7)
GAGCCTAGGGCAAGGGGAGAGCCAAAAAAAAAAAAAAAAAAAAAAp
   (233-255, plus a 22 bp polyA tail, p=phosphorylated) (SEQ ID NO:8)
AGCCTATAGCCTAGGCAAACGGCAAAAAAAAAAAAAAAAAAAAAAp
   (73-95, plus a 22 bp polyA tail, p=phosphorylated) (SEQ ID NO:9)

### Example 2

### Detection of HAV RNA by TaqMan^{™}

TaqMan^{™} technology was used for amplifying the captured target RNA. For this, amplification oligonucleotides consisted of a HAV-specific primer. The primers were as follows:

Amplification primers and detection probes in the 5' untranslated region:
VHAV1-GGATTGATTGTCAGGGCTGTC (Sense Primer-nt538-558) (Seq ID No.: 1)
VHAV2-CCCTCTCACAGGATCCCATTT (Anti-sense Primer-nt612-632, reverse complementary) (Seq ID No.: 2)
VHAV3-XCCTCTCTGTGCTTAGGGCAAACACCATTTZ (Probe-nt576-605) (Seq ID No.: 3)
   where X = 6-FAM (fluorescein), and Z = linker plus TAMRA (tetramethylrhodamine).

The nucleic acid from Example 1 was diluted to obtain about 100 IU/20 µl. Reagents for the TaqMan^{™} analysis were obtained from Applied Biosystems, Foster City, CA. The TaqMan^{™} reaction mix in a final volume of 50 ml contained: 25 ml of TaqMan^{™} One step RT-PCR Mix, 0.5 pmol of each of the amplification primers, and 0.2 pmol of the probe. The reaction conditions included 30 min at 48 °C for RT activity, 10 min at 96 °C to activate the enzyme followed by 45 cycles of 30 seconds at 95 °C, alternating with 30 seconds at 60 °C in ABI 7900 Sequence Detector. The PCR amplification sense primer VHAV1, anti-sense primer VHAV2, and probe VHAV3 were used.

An internal control transcript of 721 nts, Figure 4B (SEQ ID NO:17), which can be captured and amplified but with an altered probe-binding sequence, was prepared. The bolded letters in the sequence depicted in Figure 4B represent the sequence in the IC that replaces the sequence in the target (Figure 4A, SEQ ID NO:16). Exemplary probe sequences for the IC are xCAGTGACATGCAGGTCTAGCTz (SEQ ID NO:18) or xCCCAGTGACATGCAGGTCTAGCTz (SEQ ID NO:19) where x = TET and z = linker + TAMRA.

### Example 3

### Testing Amplification Efficiency and Capture Oligonucleotide Combinations

The 5' UTR nucleotide sequence of HAV was synthetically constructed based on the sequence ofNCBI Accession No. K02990. the sequence was cloned into M13 plasmids to provide single-stranded DNA and the DNA was purified.

### (a) Amplification efficiency.

The concentration of the cloned and purified DNA was spectrophotometrically determined and dilutions of DNA corresponding to 10,000 to 0.5 Cps per reaction were amplified in the TaqMan^{™} assay and detected using the methods, primers and probes described above. Typically, signals from samples realized <45 cycles at a threshold of >0.2 were considered positive. Table 1 details the results.

**Table 1**

| cps/rxn | Cycle 45 |
|---|---|
| 0.5 cp | 0.157663 |
| 1 cp | 0.299065 |
| 5 cp | 0.8231 |
| 10 cp | 1.115975 |
| 50 cp | 1.34539 |
| 100 cp | 1.13805 |
| 500 cp | 2.361416 |
| 1000 cp | 2.478576 |
| 5000 cp | 2.815369 |
| 10000 cp | 2.887422 |
| negative | 0.072094 |
| negative | 0.04076 |

### (b) Capture oligonucleotide combinations.

The efficiency of capture/primer combinations was tested using 25 Cps/reaction ssDNA. The combination of Capture oligonucleotides comprising the sequences of SEQ ID NOS:4, 5, 6, 7, 8 and 9 was the most efficient.

Accordingly, novel HAV sequences and detection assays using these sequences have been disclosed. From the foregoing, it will be appreciated that, although specific embodiments of the invention have been described herein for purposes of illustration, various modifications may be made without deviating from the scope of the claims.

## Claims

1. A method of detecting Hepatitis A virus (HAV) in a biological sample, the method comprising:
isolating nucleic acids from a biological sample suspected of containing HAV, wherein the nucleic acids are isolated from the biological sample by a method comprising:
i. contacting a solid support comprising capture nucleic acids associated therewith with a biological sample under hybridizing conditions wherein target nucleic acid strands hybridize with the capture nucleic acids; and
ii. separating the solid support from the sample,
and wherein the capture nucleic acids comprise one or more oligonucleotides, wherein each of the oligonucleotides is not more than about 60 nucleotides in length and comprises at least 10 contiguous nucleotides from a sequence selected from the group consisting of SEQ ID NO:10, SEQ ID NO:11, SEQ ID NO: 12, SEQ ID NO:13 and SEQ ID NO:14;
wherein the solid support comprises beads;
amplifying the isolated nucleic acids using at least two primers derived from the 5' UTR of the HAV genome,
wherein each of the primers is from 10 to 60 nucleotides in length and is sufficiently complementary to a portion of the sense and antisense strands, respectively, of the isolated nucleic acid to hybridize therewith,
and wherein
(a) one of the primers comprises a nucleotide sequence of at least 10 contiguous nucleotides from SEQ ID NO: 1 and the other primer comprises a nucleotide sequence of at least 10 contiguous nucleotides from SEQ ID NO:2, or
(b) the primers have 90% sequence identity to the nucleotide sequences described in (a); and
detecting the presence of the amplified nucleic acids as an indication of the presence or absence of HAV in the sample.

2. The method of claim 1, wherein the beads are magnetic beads.

3. The method of claim 2, wherein the isolating, amplifying and detecting are performed in a single container.

4. The method of claim 3, herein the capture nucleic acids further comprise a homopolymer chain at either the 3' or 5' end of about 15-25 nucleotides in length, wherein the homopolymer chain is selected from the group consisting of polyA, polyT, polyG, polyC, and polyU.

5. The method of claim 4, wherein the homopolymer chain is a polyA chain.

6. The method of claim 1, wherein amplifying comprises PCR, transcription-mediated amplification (TMA) or TaqMan.

7. The method of claim 6, wherein amplifying comprises TMA.

8. The method of claim 6, further comprising using a probe oligonucleotide comprising a detectable label for detecting the amplified sequence, wherein the probe oligonucleotide is not more than about 60 nucleotides in length and comprises at least 10 contiguous nucleotides comprising SEQ ID NO:3.

9. The method of claim 8, wherein the probe comprises detectable labels at the 5'-end and at the 3'-end.

10. The method of claim 9, wherein the detectable label is a fluorescent label selected from the group consisting of 6-carboxyfluorescein (6-FAM), tetramethyl rhodamine (TAMRA), and 2', 4', 5', 7',- tetrachloro -4-7- dichlorofluorescein (TET).

11. A kit for detecting Hepatitis A virus (HAV) infection in a biological sample, the kit comprising:
capture nucleic acids comprising one or more oligonucleotides, wherein each of the oligonucleotides is not more than about 60 nucleotides in length and comprises a nucleotide sequence of at least 10 contiguous nucleotides of a sequence selected from the group consisting of SEQ ID NO: 10, SEQ ID NO:11, SEQ ID NO:12, SEQ ID NO: 13 and SEQ ID NO: 14;
at least two primers wherein (a) each of the primers is not more than about 60 nucleotides in length and one primer comprises a nucleotide sequence of at least 10 contiguous nucleotides from SEQ ID NO:1 and the other primer comprises a nucleotide sequence of at least 10 contiguous nucleotides from SEQ ID NO:2;
written instructions for identifying HAV infection; and
a solid support comprising beads.

12. The kit of claim 11, further comprising a polymerase and buffers.

13. The kit of claim 11, further comprising a probe oligonucleotide of not more than about 60 nucleotides in length and at least 10 contiguous nucleotides comprising SEQ ID NO:3.

14. The kit of claim 13, wherein the probe further comprises detectable labels at the 5'-end and at the 3'-end.

15. The kit of claim 14, wherein the detectable label is a fluorescent label selected from the group consisting of 6-carboxyfluorescein (6-FAM), tetramethyl rhodamine (TAMRA), and 2', 4', 5', 7',- tetrachloro -4-7- dichlorofluorescein (TET).

## Patentansprüche

1. Verfahren zum Nachweis von Hepatitis-A-Virus (HAV) in einer biologischen Probe, bei dem man:
Nukleinsäuren aus einer biologischen Probe, von der vermutet wird, dass sie HAV enthält, isoliert,
wobei die Nukleinsäuren durch ein Verfahren aus der biologischen Probe isoliert werden, bei dem man:
i. einen festen Träger, der damit assoziierte Capture-Nukleinsäuren umfasst, unter Hybridisierungsbedingungen mit einer biologischen Probe in Kontakt bringt, wobei Target-Nukleinsäurestränge mit den Capture-Nukleinsäuren hybridisieren, und
ii. den festen Träger von der Probe trennt, und wobei die Capture-Nukleinsäuren ein oder mehrere Oligonukleotide umfassen, wobei die Oligonukleotide jeweils nicht mehr als etwa 60 Nukleotide lang sind und mindestens 10 aufeinanderfolgende Nukleotide von einer aus der aus SEQ ID NR: 10, SEQ ID NR: 11, SEQ ID NR: 12, SEQ ID NR: 13 und SEQ ID NR: 14 bestehenden Gruppe ausgewählten Sequenz umfassen,
wobei der feste Träger Kügelchen umfasst, die isolierten Nukleinsäuren unter Verwendung von mindestens zwei aus dem 5'-UTR des HAV-Genoms gewonnenen Primern amplifiziert,
wobei die Primer jeweils eine Länge von 10 bis 60 Nukleotiden aufweisen und mit einem Abschnitt des Sense- beziehungsweise Antisense-Stranges der isolierten Nukleinsäure jeweils ausreichend komplementär sind, um damit zu hybridisieren,
und wobei
(a) einer der Primer eine Nukleotidsequenz von mindestens 10 aufeinanderfolgenden Nukleotiden aus SEQ ID NR: 1 umfasst und der andere Primer eine Nukleotidsequenz von mindestens 10 aufeinanderfolgenden Nukleotiden aus SEQ ID NR: 2 umfasst oder
(b) die Primer 90% Sequenzidentität zu den in (a) beschriebenen Nukleotidsequenzen haben und
das Vorhandensein der amplifizierten Nukleinsäuren als Indikation für das Vorhandensein oder Fehlen von HAV in der Probe nachweist.

2. Verfahren nach Anspruch 1, wobei es sich bei den Kügelchen um magnetische Kügelchen handelt.

3. Verfahren nach Anspruch 2, wobei das Isolieren, das Amplifizieren und der Nachweis in einem einzelnen Behältnis erfolgen.

4. Verfahren nach Anspruch 3, wobei die Capture-Nukleinsäuren weiterhin entweder am 3'- oder am 5'-Ende eine Homopolymerkette mit einer Länge von etwa 15-25 Nukleotiden umfassen, wobei die Homopolymerkette aus der aus PolyA, PolyT, PolyG, PolyC und PolyU bestehenden Gruppe ausgewählt ist.

5. Verfahren nach Anspruch 4, wobei es sich bei der Homopolymerkette um eine PolyA-Kette handelt.

6. Verfahren nach Anspruch 1, wobei die Amplifikation PCR, Transcription-Mediated Amplification (TMA) oder TaqMan umfasst.

7. Verfahren nach Anspruch 6, wobei die Amplifikation TMA umfasst.

8. Verfahren nach Anspruch 6, bei dem man weiterhin eine Oligonukleotidsonde verwendet, welche einen nachweisbaren Marker zum Nachweis der amplifizierten Sequenz umfasst, wobei die Oligonukleotidsonde eine Länge von nicht mehr als etwa 60 Nukleotiden aufweist und mindestens 10 aufeinanderfolgende, die SEQ ID NR: 3 umfassende Nukleotide umfasst.

9. Verfahren nach Anspruch 8, wobei die Sonde am 5'-Ende und am 3'-Ende nachweisbare Marker umfasst.

10. Verfahren nach Anspruch 9, wobei es sich bei dem nachweisbaren Marker um einen aus der aus 6-Carboxyfluorescein (6-FAM), Tetramethylrhodamin (TAMRA) und 2',4',5',7'-Tetrachloro-4,7-dichlorfluorescein (TET) bestehenden Gruppe ausgewählten Fluoreszenzmarker handelt.

11. Kit zum Nachweis einer Hepatitis-A-Virus(HAV)-Infektion in einer biologischen Probe, wobei das Kit Folgendes umfasst:
Capture-Nukleinsäuren, welche ein oder mehrere Oligonukleotide umfassen, wobei die Oligonukleotide jeweils eine Länge von nicht mehr als etwa 60 Nukleotiden aufweisen und eine Nukleotidsequenz von mindestens 10 aufeinanderfolgenden Nukleotiden einer aus der aus SEQ ID NR: 10, SEQ ID NR: 11, SEQ ID NR: 12, SEQ ID NR: 13 und SEQ ID NR: 14 bestehenden Gruppe ausgewählten Sequenz umfassen,
mindestens zwei Primer, wobei (a) die Primer jeweils eine Länge von nicht mehr als etwa 60 Nukleotiden aufweisen und ein Primer eine Nukleotidsequenz von mindestens 10 aufeinanderfolgenden Nukleotiden aus SEQ ID NR: 1 umfasst und der andere Primer eine Nukleotidsequenz von mindestens 10 aufeinanderfolgenden Nukleotiden aus SEQ ID NR: 2 umfasst,
eine schriftliche Anleitung zum Identifizieren einer HAV-Infektion und
einen Kügelchen umfassenden festen Träger.

12. Kit nach Anspruch 11, welches weiterhin eine Polymerase und Puffer umfasst.

13. Kit nach Anspruch 11, welches weiterhin eine Oligonukleotidsonde mit einer Länge von nicht mehr als etwa 60 Nukleotiden und mindestens 10 aufeinanderfolgenden, SEQ ID NR: 3 umfassenden Nukleotiden umfasst.

14. Kit nach Anspruch 13, wobei die Sonde weiterhin am 5'-Ende und am 3'-Ende nachweisebare Marker umfasst.

15. Kit nach Anspruch 14, wobei es sich bei dem nachweisbaren Marker um einen aus der aus 6-Carboxyfluorescein (6-FAM), Tetramethylrhodamin (TAMRA) und 2',4',5',7'-Tetrachlor-4,7-dichlorfluorescein (TET) bestehenden Gruppe ausgewählten Fluoreszenzmarker handelt.

## Revendications

1. Procédé de détection du virus de l'hépatite A (VHA) dans un échantillon biologique, le procédé comprenant :
l'isolement d'acides nucléiques à partir d'un échantillon biologique suspecté de contenir le VHA, les acides nucléiques étant isolés de l'échantillon biologique par un procédé comprenant :
i. la mise en contact d'un support solide comprenant des acides nucléiques de capture qui lui sont associés, avec un échantillon biologique dans des conditions d'hybridation dans lesquelles les brins des acides nucléiques cibles s'hybrident aux acides nucléiques de capture ; et
ii. la séparation du support solide de l'échantillon,
et où les acides nucléiques de capture comprennent un ou plusieurs oligonucléotides, chacun des oligonucléotides ayant une longueur non supérieure à environ 60 nucléotides et comprenant au moins 10 nucléotides contigus provenant d'une séquence choisie dans le groupe consistant en SEQ ID NO:10, SEQ ID NO:11, SEQ ID NO:12, SEQ ID NO:13 et SEQ ID NO:14 ;
le support solide comprenant des perles ;
l'amplification des acides nucléiques isolés par utilisation d'au moins deux amorces dérivant de la 5'-UTR du génome du VHA,
chacune des amorces ayant une longueur de 10 à 60 nucléotides et suffisamment complémentaire d'une portion respectivement du brin sens et du brin antisens de l'acide nucléique isolé pour s'y hybrider,
et dans lequel
(a) l'une des amorces comprend une séquence nucléotidique d'au moins 10 nucléotides contigus provenant de SEQ ID NO:1, et l'autre amorce comprend une séquence nucléotidique d'au moins 10 nucléotides contigus provenant de SEQ ID NO:2, ou
(b) les amorces ont une identité de séquence de 90 % avec les séquences nucléotidiques décrites en (a) ; et
la détection de la présence des acides nucléiques amplifiés, en tant qu'indication de la présence ou de l'absence du VHA dans l'échantillon.

2. Procédé de la revendication 1, dans lequel les perles sont des perles magnétiques.

3. Procédé de la revendication 2, dans lequel l'isolement, l'amplification et la détection sont réalisés dans un récipient unique.

4. Procédé de la revendication 3, dans lequel les acides nucléiques de capture comprennent en outre une chaîne homopolymère au niveau de l'extrémité 3' ou 5', ayant une longueur d'environ 15-25 nucléotides, dans lequel la chaîne homopolymère est choisie dans le groupe consistant en polyA, polyT, polyG, polyC et polyU.

5. Procédé de la revendication 4, dans lequel la chaîne homopolymère est une chaîne polyA.

6. Procédé de la revendication 1, dans lequel l'amplification comprend une PCR, une amplification médiée par la transcription (TMA) ou un TaqMan.

7. Procédé de la revendication 6, dans lequel l'amplification comprend une TMA.

8. Procédé de la revendication 6, comprenant en outre l'utilisation d'un oligonucléotide sonde comprenant un marqueur détectable pour détecter la séquence amplifiée, dans lequel l'oligonucléotide sonde a une longueur non supérieure à environ 60 nucléotides et comprend au moins 10 nucléotides contigus comprenant SEQ ID NO:3.

9. Procédé de la revendication 8, dans lequel la sonde comprend des marqueurs détectables au niveau de l'extrémité 5' et de l'extrémité 3'.

10. Procédé de la revendication 9, dans lequel le marqueur détectable est un marqueur fluorescent choisi dans le groupe consistant en la 6-carboxyfluorescéine (6-FAM), la tétraméthylrhodamine (TAMRA) et la 2',4',5',7'-tétrachloro-4,7-dichlorofluorescéine (TET).

11. Trousse pour détecter une infection par le virus de l'hépatite A (VHA) dans un échantillon biologique, la trousse comprenant :
des acides nucléiques de capture comprenant un ou plusieurs oligonucléotides, chacun des oligonucléotides ayant une longueur non supérieure à environ 60 nucléotides et comprenant une séquence nucléotidique d'au moins 10 nucléotides contigus d'une séquence choisie dans le groupe consistant en SEQ ID NO:10, SEQ ID NO:11, SEQ ID NO:12, SEQ ID NO:13 et SEQ ID NO:14 ;
au moins deux amorces, où (a) chacune des amorces a une longueur non supérieure à environ 60 nucléotides, et une amorce comprend une séquence nucléotidique d'au moins 10 nucléotides contigus provenant de SEQ ID NO:1, et l'autre amorce comprend une séquence nucléotidique d'au moins 10 nucléotides contigus provenant de SEQ ID NO:2 ;
des instructions écrites pour identifier une infection à VHA ; et
un support solide comprenant des perles.

12. Trousse de la revendication 11, comprenant en outre une polymérase et des tampons.

13. Trousse de la revendication 11, comprenant en outre un oligonucléotide sonde ayant une longueur non supérieure à environ 60 nucléotides et au moins 10 nucléotides contigus comprenant SEQ ID NO:3.

14. Trousse de la revendication 13, dans laquelle la sonde comprend en outre des marqueurs détectables au niveau de l'extrémité 5' et de l'extrémité 3'.

15. Trousse de la revendication 14, dans laquelle le marqueur détectable est un marqueur fluorescent choisi dans le groupe consistant en la 6-carboxyfluorescéine (6-FAM), la tétraméthylrhodamine (TAMRA), et la 2',4',5',7'-tétrachloro-4,7-dichlorofluorescéine (TET).
